# EUROPÄISCHE PATENTSCHRIFT

(11) **EP 2 760 488 B1**
(45) Veröffentlichungstag und Bekanntmachung des Hinweises auf die Patenterteilung: **03.06.2015**
(21) Anmeldenummer: 12762594.5
(22) Anmeldetag: 24.09.2012
(51) Int. Cl.: A61L 15/26, A61L 15/42, C08G 18/10, C08G 18/48, C08G 18/28, C08L 75/04

(54) **SCHNELLHÄRTENDE ALKOXYSILAN-SPRÜHSCHÄUME**
FAST-SETTING ALKOXYSILANE SPRAY FOAMS
MOUSSE À PULVÉRISER ALKOXYSILANE À DURCISSAGE RAPIDE

(30) Priorität: 29.09.2011 EP 11183212
(43) Veröffentlichungstag der Anmeldung: 06.08.2014
(73) Patentinhaber: Bayer Intellectual Property GmbH, 40789 Monheim am Rhein (DE)
(72) Erfinder: SCHÖNBERGER, Jan, 42781 Haan (DE); DÖRR, Sebastian, 40593 Düsseldorf (DE)
(74) Vertreter: BIP Patents
(86) Internationale Anmeldenummer: PCT/EP2012/068793
(87) Internationale Veröffentlichungsnummer: WO 2013/045403

(56) Entgegenhaltungen:
- EP-A1- 1 829 908
- EP-A1- 2 014 314
- WO-A1-00/04069
- WO-A1-2004/104078
- WO-A1-2010/083953

## Beschreibung

Die vorliegende Erfindung betrifft ein isocyanatfreies Mehrkomponentensystem, insbesondere zur Herstellung von Schäumen für medizinische Produkte wie Wundauflagen. Die Erfindung betrifft zudem einen Formkörper, der aus einem solchen Mehrkomponentensystem erhältlich ist sowie eine Mehrkammerdruckdose, die mit einem solchen Mehrkomponentensystem befüllt ist.

Sprühbare Mehrkomponentensysteme sind aus dem Stand der Technik bekannt. So existieren sprühbare Montageschäume, die zum Ausfüllen von Hohlräumen beispielsweise im Baubereich eingesetzt werden. Sie werden insbesondere zum Auffüllen von Fugen und Hohlräumen zwischen Fensterrahmen und Türzargen und dem umliegenden Mauerwerk verwendet und zeichnen sich durch gute feuchtigkeitsisolierende Eigenschaften sowie gute Wärmedämmeigenschaften aus. Weitere Anwendungsgebiete solcher sprühbaren Mehrkomponentensysteme sind die Verwendung zur Isolierung von Rohrleitungen oder das Ausschäumen von Hohlräumen in technischen Geräten.

Typischerweise handelt es sich bei diesen vorgenannten Montageschäumen um Polyurethanschäume (PU-Schäume). Die diesen Schäumen zugrundeliegenden Zusammensetzungen bestehen aus unvernetzten Präpolymeren, die über eine hohe Anzahl freier Isocyanatgruppen verfügen. Die freien Isocyanatgruppen sind sehr reaktiv und können bereits bei normaler Umgebungstemperatur unter Einfluss von Wasser bzw. Feuchtigkeit miteinander reagieren und ein polymeres Netzwerk aus den Präpolymeren aufbauen. Neben der Luftfeuchtigkeit dienen auch Alkohole mit zwei oder mehr OH-Gruppen, entsprechende Thiole sowie primäre oder sekundäre Amine sowie Mischungen von diesen als mögliche Reaktionspartner für die vorbezeichneten Isocyanate. Besonders häufig werden hierfür Polyole verwendet. Bei der Reaktion mit Polyolen bzw. Wasser entstehen Urethan- bzw. Harnstoffeinheiten, die aufgrund ihrer Fähigkeit zur Ausbildung von Wasserstoffbrückenbindungen teilkristalline Strukturen im ausgehärteten Schaum ausbilden können. Hierdurch wird die Härte der Schäume sowie deren Druck- und Reißfestigkeit erhöht.

Die Mehrkomponentensysteme werden häufig in Druckdosen eingefüllt und mit einem Treibmittel versehen, das das Aufschäumen der Präpolymere beim Ausbringen aus der Druckdose erleichtert. Zudem reagieren die Isocyanatgruppen des Präpolymers mit der Luftfeuchtigkeit, wobei Kohlendioxid abgespalten wird, welches ebenfalls zum Aufschäumen beiträgt. Bei dieser Reaktion werden die beteiligten Isocyanatgruppen zu Aminen umgewandelt, die ihrerseits mit weiteren Isocyanatgruppen unter Ausbildung eines polymeren Netzwerks reagieren können, mit anderen Worten also der Vernetzungsreaktion nicht verloren gehen.

Die Polyurethanzusammensetzungen können als 1K-Schäume oder auch als zweikomponentige (2K) Schäume hergestellt werden. Während die 1K-Schäume nur durch den Einfluss von Luftfeuchtigkeit aushärten, werden bei den 2K-Schäumen eine Isocyanatkomponente und eine Polyolkomponente getrennt voneinander aufbewahrt und erst unmittelbar vor dem Ausbringen miteinander vermischt. Der Mischvorgang geschieht hierbei entweder in dem Druckkörper der Druckdose, deren Inhalt dann zügig vollständig aufgebraucht werden muss, da die Polymerisationsreaktion unabhängig davon stattfindet, ob der Schaum ausgebracht wird oder nicht. Solche Systeme werden daher häufig auch als 1,5K-Schäume bezeichnet.

Eine andere Möglichkeit besteht darin, eine Zweikammerdruckdose zu verwenden, in der die beiden Komponenten erst im Bereich des Auslassventils miteinander vermischt werden. Der Hauptvorteil der 2K-Schäume gegenüber den 1K-Schäumen besteht in der erheblich schnelleren Aushärtungsreaktion, da diese auch in Abwesenheit von Luftfeuchtigkeit stattfindet. Demgegenüber ist die Aushärtegeschwindigkeit bei 1K-Schäumen durch die Luftfeuchtigkeit bedingt sowie auch durch die Diffusionsgeschwindigkeit der Luftfeuchtigkeit in das ausgeschäumte Material.

Die vorgenannten Mehrkomponentensysteme enthalten typischerweise neben den Präpolymerkomponenten noch weitere Hilfsstoffe, wie beispielsweise Schaumstabilisatoren sowie Katalysatoren, die die Vernetzungsreaktion beschleunigen sollen. Für letztere werden in erster Linie titan- oder zinnorganische Verbindungen verwendet, wie beispielsweise Dibutylzinndilaurat.

Die vorbezeichneten Polyurethanschäume besitzen im ausgehärteten Zustand gute mechanische und wärmedämmende Eigenschaften und haften auf den meisten Untergründen sehr gut. Allerdings können die vorbezeichneten Polyurethanschäume noch monomere Diisocyanate enthalten, was bei einer Verwendung der Schäume zur Behandlung von Wunden unerwünscht ist.

Um das Gefährdungspotential der vorgenannten Sprühschäume zu reduzieren, werden in DE 43 03 848 A1 Präpolymere beschrieben, die keine monomeren Isocyanate beinhalten bzw. allenfalls nur geringe Konzentrationen an diesen Verbindungen aufweisen. Hier besteht jedoch ein gewisses Risiko, dass das Präpolymer noch freie Isocyanatgruppen aufweisen kann, was wiederum für medizinische Anwendungen unerwünscht ist.

Aus den vorgenannten Gründen wurden in den letzten Jahren polymerisierbare schäumbare Zusammensetzungen entwickelt, welche nicht über freie Isocyanatgruppen aushärten. So sind beispielsweise aus der US 6,020,389 A1 Silikonschäume bekannt, welche Alkoxy-, Acyloxy- oder Oximo-terminierte Silikonpräpolymere enthalten. Diese Verbindungen polymerisieren über eine Kondensationsreaktion von Siloxangruppen. Nachteilig bei diesen Verbindungen ist ihre lange Aushärtezeit, da sie - wie auch die 1K-Polyurethan-Sprühschäume - auf Luftfeuchtigkeit für die Polymerisationsreaktion angewiesen sind. Insbesondere bei dickeren ausgeschäumten Schichten benötigt die vollständige Abreaktion entsprechend viel Zeit. Dies ist nicht nur unkomfortabel, sondern auch insofern problematisch, als dass die durch das Aussprühen gebildete Schaumstruktur zum Teil wieder zusammenfällt, bevor die Porenwände durch die fortschreitende Polymerisationsreaktion eine ausreichende Eigenfestigkeit aufbauen können.

Aus der WO 00/04069 sind Alkoxysilan-terminierte Polyurethanpräpolymere bekannt. Diese Präpolymere besitzen ein herkömmliches Polyurethanrückgrat, auch als "backbone" bezeichnet, welches auf an sich bekannte Weise durch Umsetzung von difunktionellen Isocyanaten mit Polyolen erhalten wird. Durch Einsatz eines Überschusses von polyfunktionellen Isocyanaten wird erreicht, dass die jeweiligen Endgruppen der Präpolymerketten freie Isocyanatgruppen besitzen. Diese Isocyanat-terminierten Präpolymere werden dann in einem weiteren Reaktionsschritt mit einem Aminoalkyltrialkoxysilan zu den gewünschten Alkoxysilan-terminierten Polyurethanpräpolymeren umgesetzt. Insbesondere wird hierzu Aminopropyltrimethoxisilan verwendet. Das hieraus erhaltene Präpolymer trägt Trimethoxisilan-terminierte Endgruppen, die über einen Propylen-spacer an das Polyurethanrückgrat angekoppelt sind. Aufgrund der Propylengruppe zwischen dem Siliciumatom und dem Polyurethan-Backbone werden solche Silane auch als γ-Silane bezeichnet.

Bei der Aushärtungsreaktion reagieren die γ-Silane unter Einwirkung von Wasser unter Alkoholabspaltung und bilden hierbei Si-O-Si-Netzwerke aus, wodurch das Präpolymer aushärtet. Die γ-Silane weisen ebenso wie die Isocyanat-terminierten Polyurethan-Präpolymere den Nachteil auf, dass die Aushärtungsreaktion vergleichsweise langsam abläuft. Dieser Nachteil kann nur zum Teil dadurch kompensiert werden, dass γ-Silan-basierte Zusammensetzungen große Mengen an Vernetzungskatalysatoren zugesetzt werden, wie beispielsweise das auch für Polyurethan-Präpolymere verwendete Dibutylzinndilaurat. Dies wirkt sich jedoch zum Teil nachteilig auf die Lagerstabilität solcher Zusammensetzungen aus.

Da auch größere Mengen an Vernetzungskatalysator die Reaktionsträgkeit der γ-Silane nicht vollständig kompensieren können, wurde nach reaktiveren Verbindungstypen geforscht. Solche sind beispielsweise aus der WO 02/066532 A1 bekannt. Auch bei den hier beschriebenen Präpolymeren handelt es sich um Silan-terminierte Polyurethan-Präpolymere. Der wesentliche Unterschied zu den zuvor beschriebenen γ-Silanen besteht darin, dass zwischen dem Polyurethan-Backbone und dem Siliciumatom anstelle der Propylen-Gruppe ein Methylen-Spacer eingesetzt ist. Aufgrund dessen werden diese Silane auch als α-Silane bezeichnet. Der kürzere Abstand des Siliciumatoms zu der stark polaren Harnstoffgruppe des Polyurethan-backbones erhöht die Reaktivität der an dem Siliciumatom befindlichen Alkoxygruppen (α-Effekt), so dass die Hydrolyse der Alkoxysilangruppen und die anschließende Kondensationsreaktion mit erheblich gesteigerter Geschwindigkeit abläuft.

Nachteilig sowohl bei α-Silanen als auch bei γ-Silanen ist jedoch, dass die Herstellung sprühbarer Schäume aus diesen Präpolymeren äußerst schwierig ist. Insbesondere die Bereitstellung eines in Druckdosen abfüllbaren Sprühschaums, der eine lockere Porenstruktur mit großem Porenvolumen erzeugen können soll, ist kaum zu bewerkstelligen. Der Grund hierfür ist, dass bei der Vernetzungsreaktion im Gegensatz zu den Polyurethan-Schäumen bei Einwirkung von Wasser keine gasförmigen Reaktionsprodukte (wie CO₂ beim Polyurethanschaum) entstehen, sondern Alkohole abgespalten werden, wie beispielsweise Methanol oder Ethanol. Diese Verbindungen sind jedoch im Gegensatz zu einem gasförmigen Reaktionsprodukt nicht in der Lage, aufschäumende Wirkungen zu entfalten, so dass ein aus einer Druckdose ausgesprühter Schaum bis zum Aushärten stark in sich zusammenfällt. Diesem Effekt kann auch mit dem Einsatz von Schaumstabilisatoren nur bedingt entgegnet werden.

Mit diesem Problem befasst sich die EP 1 829 908 A1, in der ein 2K-Silanpräpolymer basierendes System vorgeschlagen wird. Hierbei wird in einer ersten Komponente das Silan-Präpolymer, wie beispielsweise ein silanterminiertes-Polyurethan-Präpolymer, Dibutylzinndilaurat als Katalysator sowie größere Mengen als Calciumcarbonat verwendet. Die zweite Komponente besteht aus einer hochkonzentrierten wässrigen Zitronensäurelösung. Zum Ausschäumen dieses 2K-Schaumes werden die beiden Komponenten miteinander vermischt und über einen Sprayapplikator an gewünschter Stelle ausgebracht. Dabei bewirkt das in der zweiten Komponente enthaltene Wasser die Vernetzungsreaktion des Silan-Präpolymers, wobei das Calciumcarbonat unter Einwirkung der hochkonzentrierten Zitronensäurelösung CO₂ freisetzt. Das Kohlendioxid bewirkt - wie von den Polyurethan-Präpolymeren bekannt - ein Aufschäumen der ausgebrachten Präpolymermischung.

Nachteilig bei diesem System ist jedoch, dass die hochkonzentrierte Zitronensäurelösung einen pH-Wert von etwa 1-2 besitzt und aufgrund dessen ätzende oder zumindest reizende Eigenschaften entfaltet. Insbesondere beim Aussprühen aus Druckdosen kann es zur Aerosolbildung kommen, wodurch die Augen, die Haut und insbesondere auch die Atemwege des Anwenders gereizt werden. Zudem schränkt das ätzende bzw. korrosive Potential der Zitronensäure den Anwendungsbereich der Zusammensetzungen erheblich ein. So ist es beispielsweise undenkbar, solche Zusammensetzungen im medizinischen Bereich als sprühbare Wundauflage unmittelbar auf der Haut, insbesondere einer verletzten Hautstelle bzw. einem verletzten Körperteil aufzubringen.

Vor diesem Hintergrund besteht die Aufgabe der vorliegenden Erfindung darin, ein Mehrkomponentensystem der eingangs genannten Art dahingehend zu modifizieren, dass hieraus Sprühschäume hergestellt werden können, welche schnell aushärten, eine stark poröse Struktur mit einem hohen Porenvolumen aufweisen und ein breiteres Anwendungsgebiet ermöglichen. Zudem soll die Zusammensetzung ein möglichst geringes Gefahrenpotential für den Anwender darstellen und insbesondere auch zur Herstellung von sprühbaren Wundauflagen geeignet sein.

Diese Aufgabe wird durch ein isocyanatfreies Mehrkomponentensystem, insbesondere zur Herstellung von Schäumen für medizinische Produkte wie Wundauflagen, mit wenigstens zwei getrennten Komponenten, wobei die erste Komponente zumindest ein Alkoxysilan-terminiertes Präpolymer und die zweite Komponente eine wässrige Komponente umfasst, wobei die wässrige Komponente eine Polyurethandispersion umfasst, gelöst.

Unter isocyanatfrei wird vorliegend ein System verstanden, das weniger als 0,5 Gew.-% isocyanathaltige Komponenten enthält.

Überraschenderweise hat sich herausgestellt, dass durch Verwendung einer wässrigen Polyurethandispersion ein solches Mehrkomponentensystem in eine Mehrkammerdruckdose abgefüllt werden kann, wobei die Polyurethandispersion dazu führt, dass sich eine Reihe von handelsüblichen Treibgasen in der wässrigen Phase lösen. Hierdurch wird eine Phasenseparation von Treibgas und zweiter Komponente verhindert. Zudem ist die Löslichkeit dieser Treibgase in der das Präpolymer enthaltenden ersten Komponente eher weniger problematisch. Insofern befinden sich das Treibgas und die erste Komponente bzw. das Treibgas und die zweite Komponente bis zum Verlassen der Druckdose in weitestgehend homogener Mischung. Nach dem Vermischen der beiden in der Dose getrennt vorgehaltenen ersten und zweiten Komponente in einer Mischdüse der Druckdose führt das in der Mischung gelöste Treibgas nach dem Verlassen der Druckdose zu einer starken Expansion dieser Mischung, so dass ein feinporiger Schaum erhalten wird. Mit anderen Worten stellt die vorliegende Erfindung ein 2K-Silanschaumsystem zur Verfügung, aus dem Polymerschäume mit hohem Porenvolumen erhältlich sind, ohne dass es den zusätzlichen Einsatz gasentwickelnder Reaktanden, wie beispielsweise die Kombination von Calciumcarbonat und Zitronensäure, erfordert.

Das erfindungsgemäße Mehrkomponentensystem ist für eine Vielzahl von Applikationen einsetzbar. So eignet es sich für sämtliche Anwendungsgebiete, in denen die vorbezeichneten Polyurethanschäume und auch α- bzw. γ-Silanschäume vorgeschlagen werden, also für den Baubereich, zur Isolation von Rohren oder auch zum Ausschäumen von Hohlräumen in Maschinen.

Überraschenderweise hat sich zudem herausgestellt, dass das erfindungsgemäße Mehrkomponentensystem auch im medizinischen Sektor verwendet werden kann, da dieses keine toxischen oder reizenden Verbindungen beinhaltet.

Der medizinische Anwendungsbereich umfasst hierbei beispielsweise die Bereitstellung in-situ herstellbarer Wundauflagen. Dazu kann das erfindungsgemäße Mehrkomponentensystem nach dem Vermischen der beiden Komponenten auf Hautverletzungen oder Verletzungen anderer Art aufgesprüht oder anderweitig aufgetragen werden. Dabei haften die ausgeschäumten Zusammensetzungen nicht merklich auf organischem Gewebe wie beispielsweise Wundgewebe und sind zudem aufgrund ihrer Porenstruktur in der Lage, Wundsekrete oder Blut aufzusaugen. Dies ist wohl darin begründet, dass das erfindungsgemäße Mehrkomponentensystem beim Aussprühen unter den vorgenannten Bedingungen wenigstens teilweise eine offene Porenstruktur ausbildet und damit saugfähig ist.

Ein weiterer Vorteil des erfindungsgemäßen Mehrkomponentensystems zeigt sich bei den vorgenannten medizinischen Anwendungen auch dahingehend, als dass die Härte des erhaltenen Polymerschaums durch die Wahl der chemischen Natur und/oder der Kettenlänge des Polymerrückgrats des Silan-Präpolymers variabel gestaltet werden kann. Neben den vorgenannten Parametern lässt sich die Härte des Schaumes auch durch weitere Maßnahmen modifizieren, wie beispielsweise durch den Vernetzungsgrad. Es können somit sehr weiche und damit nachgebende Polymerschäume oder auch feste Polymerschäume mit stützenden Eigenschaften gebildet werden. Insofern ist der medizinische Anwendungsbereich nicht nur auf die unmittelbare Wundbehandlung beschränkt, sondern es ist auch die Ruhigstellung von Gliedmaßen, beispielsweise bei Knochenbrüchen, Bänderdehnungen, Verstauchungen und dergleichen möglich. Zudem sind ebenfalls Anwendungen im kosmetischen Bereich denkbar.

Auch wenn die Bereitstellung des erfindungsgemäßen Mehrkomponentensystems in Druckdosen eine komfortable Möglichkeit darstellt, ist die Erfindung jedoch nicht hierauf beschränkt. So lässt sich das erfindungsgemäße Mehrkomponentensystem nach dem Vermischen der beiden Komponenten auch ohne weiteres als Gießmasse verwenden.

Das erfindungsgemäß in der ersten Komponente enthaltene Silan-terminierte Präpolymer kann im Prinzip sämtliche Typen von Polymer-Backbones aufweisen sowie auch Mischungen hieraus.

Nach einer bevorzugten Ausführungsform umfasst das Alkoxysilan-terminierte Präpolymer ein Alkoxysilan-terminiertes Polyurethan-Präpolymer. Das Polyurethan-Backbone kann hierbei auf verschiedene Weisen aufgebaut sein. So ist es zum einen möglich, ein Polymerrückgrat durch Umsetzung von Diisocyanaten mit Diolen zu erzeugen, wodurch das Polymer-Backbone eine Vielzahl von innenliegenden Urethan-Gruppen aufweist. Die durch die Reaktionsführung entstehenden endständigen Isocyanatgruppen werden anschließend mit Aminoalkylalkoxysilanen umgesetzt. Auf diese Weise werden Silan-terminierte Präpolymere erhalten, die je nach Kettenlänge die Herstellung von vergleichsweise festen Schäumen erlauben.

Unter einem Polyurethan-Präpolymer wird im Sinne der vorliegenden Erfindung auch ein Polymer-Backbone verstanden, welches in seiner Hauptkette beispielsweise nur Polyether- und/oder Polyesterpolyole aufweist und das an seinen Kettenenden Isocyanatgruppen trägt. Ein solches Polymer-Backbone ist insbesondere für medizinische Anwendungen vorteilhaft, weil das entsprechende Silan-terminierte Präpolymer eine ausreichend niedrige Viskosität aufweist, so dass es sich leicht ausschäumen lässt. Demgegenüber sind Urethan- oder Harnstoffgruppen im Polymer-Backbone weniger bevorzugt, da diese die Viskosität zum Teil beträchtlich erhöhen.

Gemäß einer besonders bevorzugten Ausführungsform der Erfindung kann das Alkoxysilan-terminierte Präpolymer ein Alkoxysilan-terminiertes Polyurethanpräpolymer umfassen, welches insbesondere aus einem Aminoalkoxysilan und einem Isocyanat-terminierten Präpolymer hergestellt ist, wobei das Isocyanat-terminierte Präpolymer insbesondere aus einem Polyol und einem aliphatischen Polyisocyanat hergestellt sein kann.

Um Silan-terminierte Polyurethan-Präpolymere mit niedriger Viskosität zu erhalten, ist es zudem besonders vorteilhaft, die Isocyanat-terminierten Präpolymere herzustellen, indem ein Überschuss der Isocyanatkomponente mit der Polyolkomponente umgesetzt und anschließend der nicht umgesetzt Teil an Isocyanatkomponente bevorzugt durch Dünnschichtdestillation entfernt wird. Die Umsetzung des auf diese Weise hergestellten und gereinigten Isocyanat-terminierten Präpolymers mit Alkoxysilanen liefert besonders niedrigviskose Silan-terminierte Präpolymere.

Erfindungsgemäß einsetzbare Polyetherpolyole sind beispielsweise die in der Polyurethanchemie an sich bekannten Polytetramethylenglykolpolyether, die beispielsweise durch Polymerisation von Tetrahydrofuran mittels kationischer Ringöffnung erhältlich sind. Ebenfalls geeignet sind die an sich bekannten Additionsprodukte von Styroloxid, Ethylenoxid, Propylen¬oxid, Butylenoxide und/oder Epichlorhydrin an di- oder polyfunktionelle Startermoleküle. Als Startermoleküle können dabei alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Ein weiterer Vorteil der Polyether- bzw. Polyesterpolyole im Polymerrückgrat besteht darin, dass damit die Hydrophilie des erhaltenen Schaums bedarfsgerecht eingestellt werden kann, so dass dieser beispielsweise ein besseres Aufnahmevermögen gegenüber wässrigen Flüssigkeiten, wie Blut oder Wundsekreten entfaltet. Dabei ist es jedoch zweckmäßig, den Anteil an Ethylenoxideinheiten im Polyetherpolyol nicht zu hoch einzustellen, da dies ansonsten zu einem starken Aufquellen der Wundauflage führen würde. Insofern ist eine bevorzugte Ausführungsform des erfindungsgemäßen Mehrkomponentensystems dadurch definiert, dass der Anteil an Ethylenoxid-Bausteinen im Polyetherpolyol höchstens 50 Gew.-% beträgt, vorzugsweise höchstens 30 Gew.-%, weiter bevorzugt höchstens 20 Gew.-%. Die Untergrenze an Ethylenoxid-Gruppen kann beispielsweise bei etwa 5 Gew.-% liegen. Ungeachtet dessen können auch Polyetherpolyole ohne Ethylenoxideinheiten verwendet werden.

Was die Polyester-Einheiten betrifft, so können diese monofunktionell oder multifunktionell sein, insbesondere difunktionell.

Die im Rahmen der vorliegenden Erfindung einsetzbaren Polyetherpolyole bzw. Polyesterpolyole können aus aliphatischen Einheiten aufgebaut sein oder aber auch aromatische Gruppen besitzen. Dem gegenüber ist es in Bezug auf die eingesetzten Isocyanate bzw. multifunktionellen Isocyanate besonders bevorzugt, wenn diese nur aliphatische Gruppen aufweisen. Mit anderen Worten werden erfindungsgemäß bevorzugt keine aromatischen Isocyanate eingesetzt.

Zur Herstellung des erfindungsgemäßen Alkoxysilan-terminierten Präpolymers sind grundsätzlich die dem Fachmann an sich bekannten aromatischen, araliphatischen, aliphatischen oder cycloaliphatischen Polyisocyanate einer NCO-Funktionalität von ≥ 2 geeignet. Beispiele derartiger Polyisocyanate sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3-und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4"-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Nach einer besonders bevorzugten Ausführungsform des erfindungsgemäßen Mehrkomponentensystems umfasst das Alkoxysilan-terminierte Präpolymer α-Silangruppen. Hierbei ist ebenso vorgesehen, dass das in der erfindungsgemäßen Zusammensetzung enthaltene Alkoxysilan-terminierte Präpolymer ausschließlich α-Silangruppen aufweist.

Unter einer α-Silangruppe versteht man, wie bereits vorgehend ausgeführt wurde, dass zwischen dem Siliciumatom und dem Polymer-Backbone ein Methylen-Spacer vorhanden ist. Solche Silane zeichnen sich durch eine besondere Reaktionsfreudigkeit in Bezug auf die Kondensationsreaktion aus. Aufgrund dessen ist es im Rahmen der vorliegenden Erfindung möglich, vollständig auf den Einsatz Schwermetall-basierter Vernetzungskatalysatoren wie organische Titanate oder organische Zinn(IV)Verbindungen zu verzichten. Dies ist insbesondere bei medizinischen Anwendungsfeldern für das erfindungsgemäße Mehrkomponentensystem von Vorteil.

In weiter bevorzugter Weise handelt es sich bei den α-Silangruppen des eingesetzten Alkoxysilan-terminierten Präpolymers um Triethoxy-α-Silangruppen. Dies ist insofern von Vorteil, als dass bei der Vernetzungsreaktion vergleichsweise ungefährliches Ethanol freigesetzt wird anstelle von Methanol bei den häufig verwendeten Methoxy-α-Silanen. Auch wenn die Reaktivität und damit Aushärtegeschwindigkeit der Trimethoxy-α-Silane höher ist als diejenige der Triethoxy-α-Silane, ist die Reaktivität der Triethoxy-α-Silane ausreichend hoch, als dass die Mischung binnen weniger Minuten vollständig aushärtet, teilweise auch nach weniger als einer Minute.

Bevorzugt ist ebenfalls, wenn es sich bei den α-Silangruppen des eingesetzten Alkoxysilan-terminierten Präpolymers um Diethoxy-α-Silangruppen handelt.

In weiter bevorzugter Weise beträgt das Gewichtsmittel des Alkoxysilan-terminierten Präpolymers 500 bis 20000 g/Mol, vorzugsweise 500 bis 6000 g/Mol, insbesondere 2000 bis 5000 g/Mol. Die vorgenannten Molekulargewichte sind insbesondere in Bezug auf Polyether- und Polyesterpolyole von Vorteil, die hieraus herstellbaren ausgehärteten erfindungsgemäßen Zusammensetzungen wahlweise von sehr weich bis sehr fest eingestellt werden können. Sind beispielsweise sehr weiche ausgehärtete Zusammensetzungen gewünscht, ist es von Vorteil, wenn das Gewichtsmittel des Alkoxysilan-terminierten Präpolymers mit einem Polyetherpolyol bei mehr als 2000 g/Mol liegt, vorzugsweise bis hin zu 20000 g/ Mol, insbesondere bei wenigstens 3000 g/Mol, besonders bevorzugt bei wenigstens 3200 g/Mol, ganz besonders bevorzugt bei 3500 bis 6000 g/Mol. Für die Herstellung ausgehärteter Zusammensetzungen vergleichbarer Festigkeit ist unter Einsatz von Präpolymeren mit Polyester-polyolen ein Gewichtsmittel des Alkoxysilan-terminierten Präpolymers von bis zu 2000 g/Mol, insbesondere von 300 bis zu 1500 g/Mol sinnvoll.

Das mittlere Molekulargewicht der Polyole wird wie folgt bestimmt: Zunächst wird die OH-Zahl durch Veresterung und anschließende Rücktitration des überschüssigen Veresterungsreagenz mit alkoholischer Kaliumhydroxidmaßlösung experimentell ermittelt. Die OH-Zahl wird in mg KOH pro Gramm Polyol angegeben. Aus der OH-Zahl wird über die Formel mittleres Molekulargewicht = 56 x 1000 x OH-Funktionalität / OH-Zahl das mittlere Molekulargewicht errechnet.

Im Rahmen der vorliegenden Erfindung ist vorgesehen, dass die zweite, wässrige Komponente, eine Polyurethandispersion ist. Hierunter wird im Rahmen der vorliegenden Erfindung verstanden, dass beispielsweise eine handelsübliche Polyurethandispersion eingesetzt werden kann, deren Konzentration jedoch auch mit zusätzlichem Wasser verringert werden kann. Als Polyurethandispersion können im Prinzip sämtliche am Markt erhältliche Polyurethandispersionen verwendet werden. Jedoch ist es auch hier von Vorteil, Polyurethandispersionen einzusetzen, welche aus aromatenfreien Isocyanaten hergestellt wurden, da diese insbesondere für medizinische Anwendungen unbedenklicher sind. Zudem kann die Polyurethandispersion auch weitere Inhaltsstoffe beinhalten. In besonders bevorzugter Weise enthält die Polyurethandispersion 5 bis 65 Gew.-% Polyurethan, insbesondere 20 bis 60 Gew.-%.

In Weiterbildung des erfindungsgemäßen Mehrkomponentensystems beträgt das Gewichtsmittel des Polyurethans der Polyurethandispersion 10000 bis 1000000 g/Mol, insbesondere 20000 bis 200000 g/Mol, jeweils ermittelt über Gelpermeationschromatographie gegenüber Polystyrol-Standard in Tetrahydrofuran bei 23°C. Polyurethandispersionen mit solchen Molmassen sind besonders vorteilhaft, da diese lagerstabile Polyurethandispersionen darstellen, die zudem beim Abfüllen in Druckdosen eine gute Löslichkeit des Treibgases in der zweiten Komponente bewirken.

In weiter bevorzugter Weise besitzt die Polyurethandisperson einen pH-Wert von 5,0 bis 9,0. Dies ist besonders vorteilhaft, weil Polyurethandispersionen in diesem pH-Bereich zum einen die Kondensationsreaktion des Silan-terminierten Präpolymers beschleunigen. Zudem ist dieser bevorzugte pH-Bereich auch aus physiologischer Sicht akzeptabel, so dass solche Zusammensetzungen auch in medizinischen Anwendungsfeldern eingesetzt werden können. Für Applikationen direkt auf der Haut oder an Wunden ist es besonders vorteilhaft, einen pH-Wert für die Polyurethandispersion vorzusehen, der sich in der Nähe des pH-Werts der menschlichen Haut bewegt, also beispielsweise von 5,0 bis 7,0, insbesondere bevorzugt bei etwa pH 5,5.

Die vorgenannten pH-Werte lassen sich bei den Polyurethan-Dispersionen auf verschiedene Weisen einstellen. So kann die Polyurethan-Dispersion beispielsweise mit einer Säure, einer Base oder einem Puffer versetzt werden, wobei die jeweiligen Einsatzmengen dieser Substanzen so gewählt sind, dass der gewünschte pH-Wert erzielt wird. Ein weiterer Vorteil der vorgenannten pH-Werte besteht zudem darin, dass es in diesen Bereichen in der Regel nicht zu einer Koagulation der Polymerpartikel der Polyurethan-Dispersion kommt, mit anderen Worten die Dispersion unter diesen Bedingungen lagerstabil ist.

Nach einer besonders bevorzugten Ausführungsform der erfindungsgemäßen Zusammensetzung enthält die erste und/oder die zweite Komponente einen medizinischen und/ oder kosmetischen Wirkstoff. Zwischen diesen beiden Wirkstoffgruppen existiert keine scharfe Abgrenzung, da viele medizinische Wirkstoffe auch kosmetische Wirkungen entfalten.

In diesem Zusammenhang ist es ebenfalls denkbar, den oder die Wirkstoffe in Form einer weiteren, d.h. dritten oder vierten Komponente vorzusehen und erst unmittelbar vor der Applikation des Mehrkomponentensystems mit der ersten und zweiten Komponente zu vermischen. Aufgrund der Erhöhung der Komplexität des Mehrkomponentensystems mit zunehmender Zahl an separaten Komponenten ist dieser Weg jedoch in der Regel nur dann sinnvoll, wenn die eingesetzten Wirkstoffe sowohl mit der ersten als auch mit der zweiten Komponente unverträglich sind.

Die Wirkstoffe können als reiner Wirkstoff oder aber in verkapselter Form vorliegen, um beispielsweise eine zeitlich verzögerte Abgabe zu erzielen.

Als kosmetische Wirkstoffe kommen insbesondere solche Wirkstoffe in Betracht, welche hautpflegende Eigenschaften besitzen, beispielsweise feuchtigkeitsspendende oder hautberuhigende Wirkstoffe.

Als medizinische Wirkstoffe können im Rahmen der vorliegenden Erfindung eine Vielzahl von Wirkstofftypen und -klassen eingesetzt werden.

Ein solcher medizinischer Wirkstoff kann beispielsweise eine unter in vivo-Bedingungen Stickstoffmonoxid-freisetzende Komponente, bevorzugt L-Arginin oder eine L-Arginin-haltige oder eine L-Arginin freisetzende Komponente, besonders bevorzugt L-Arginin Hydrochlorid umfassen. Auch Prolin, Ornithin und/oder andere biogene Zwischenstufen wie beispielsweise biogene Polyamine (Spermin, Spermitin, Putrescin oder bioaktive künstliche Polyamine) können verwendet werden. Derartige Komponenten unterstützen bekanntermaßen die Wundheilung, wobei deren kontinuierliche mengenmäßig nahezu gleichmäßige Abgabe der Wundheilung besonders zuträglich ist.

Weitere erfindungsgemäß verwendbare Wirkstoffe umfassen mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Als Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam® geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF α; (Transforming Growth Factor alpha), TGF ß (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere ß-Carotin, Vitamin E, Gruppe der Tocopherole, insbesondere α Tocopherol, ß-Tocopherol, γ-Tocopherol, δ-Tocopherol und α-Tocotrienol, ß-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B 1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das gemizid, bakterizid, bakteriostatisch, fungizid, viruzid, virustatisch und/ oder allgemein mikrobiozid wirkt.

Insbesondere sind solche Stoffe geeignet die ausgewählt werden aus der Gruppe Resorcinol, lod, lod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobielle Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche, wundheilungsfördernde Wirkstoffe sind im Zusammenhang mit der vorliegenden Erfindung insbesondere Extrakte der Kamille, Hamamelis-Extrakte z.B. Hamamelis virgina, Calendula-Extrakt, Aloe- Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu nennen.

Der Gehalt der Wirkstoffe richtet sich dabei in erster Linie an der medizinisch erforderlichen Dosis aus sowie auch an der Verträglichkeit mit den übrigen Bestandteilen der erfindungsgemäßen Zusammensetzung.

Ferner können die erfindungsgemäßen Mehrkomponentensysteme auch weitere Hilfsstoffe zugesetzt werden. Hierfür kommen beispielsweise Schaumstabilisatoren, Verdicker bzw. Thixotropiermittel, Antioxidantien, Lichtschutzmittel, Emulgatoren, Weichmacher, Pigmente, Füllstoffe, Additive zur Gebindestabilisierung, Biozide, Colösemittel, und/oder Verlaufshilfsmittel in Frage.

Als Schaumstabilisatoren eignen sich beispielsweise Alkylpolyglycoside. Diese sind nach dem Fachmann an sich bekannten Methoden durch Umsetzung von längerkettigen Monoalkoholen mit Mono-, Di- oder Polysacchariden erhältlich (Kirk-Othmer Encyclopedia of Chemical Technology, John Wiley & Sons, Vol. 24, S. 29). Die längerkettigen Monoalkohole, die gegebenenfalls auch verzweigt sein können, weisen bevorzugt 4 bis 22 C-Atome, bevorzugt 8 bis 18 C-Atome und besonders bevorzugt 10 bis 12 C-Atome in einem Alkylrest auf. Im Einzelnen genannt seien als längerkettige Monoalkohole 1-Butanol, 1-Propanol, 1-Hexanol, 1-Oktanol, 2-Ethylhexanol, 1-Decanol, 1-Undecanol, 1-Dodecanol (Laurylalkohol), 1-Tetradecanol (Myristylalkohol) und 1-Octadecanol (Stearylalkohol). Selbstverständlich können auch Gemische der genannten längerkettigen Monoalkohole eingesetzt werden.

Bevorzugt weisen diese Alkylpolyglycoside von der Glucose abgeleitete Strukturen auf. Besonders bevorzugt werden Alkylpolyglycoside der Formel (I) eingesetzt.

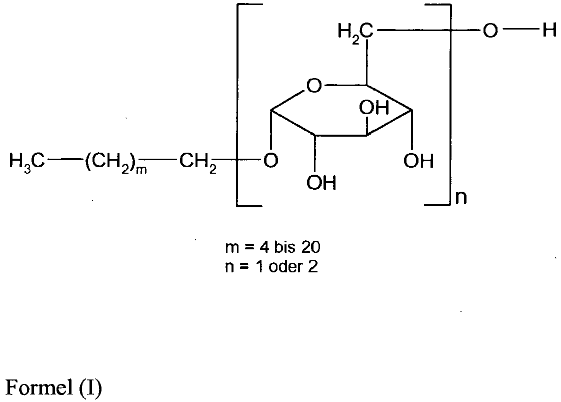

Bevorzugt ist m eine Zahl von 6 bis 20, besonders bevorzugt 10 bis 16.

Die Alkylpolyglycoside weisen bevorzugt einen HLB-Wert von weniger als 20, besonders bevorzugt von weniger als 16 und ganz besonders bevorzugt von weniger als 14 auf, wobei sich der HLB nach der Formel HLB = 20 · Mh/M errechnet, wobei Mh die Molmasse des hydrophilen Anteils eines Moleküls und M die Molmasse des gesamten Moleküls ist (Griffin, W.C.: Classification of surface active agents by HLB, J. Soc. Cosmet. Chem. 1, 1949).

Weitere Schaumstabilisatoren umfassen an sich bekannte anionische, kationische, amphotere und nichtionische Tenside sowie Mischungen hieraus. Bevorzugt werden Alkylpolyglycoside, EO/PO-Blockco¬polymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere eingesetzt.

Zudem können zur Verbesserung der Schaumeigenschaften des resultierenden Schaums an sich bekannte ein- und mehrwertige Alkohole sowie Mischungen hieraus verwendet werden. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Diese Schaumstabilisatoren können der ersten und / oder vorzugsweise der zweiten Komponente zugegeben werden, sofern keine chemische Reaktion mit den jeweiligen Komponenten auftritt. Dabei beträgt der Gesamtgehalt an diesen Verbindungen bezogen auf das erfindungsgemäße Mehrkomponentensystem insbesondere 0,1 bis 20 Gew.-%, bevorzugt 1 bis 10 Gew.-%.

Die Mengenverhältnisse der ersten und zweiten Komponente des erfindungsgemäßen Mehrkomponentensystems werden vorteilhafterweise so zueinander eingestellt, dass eine vollständige Polymerisation erfolgt und dabei die erste Komponente möglichst quantitativ umgesetzt wird. Beispielsweise liegen deshalb die erste und die zweite Komponente des erfindungsgemäßen Mehrkomponentensystems in einem Volumenverhältnis von 1:10 bis 10:1 zueinander vor, vorzugsweise in einem Volumenverhältnis von 1:1 bis 5:1 zueinander, insbesondere 2:1 bis 3:1, besonders bevorzugt bei etwa 2,5:1.

Gemäß einer besonders bevorzugten Ausführungsform der Erfindung kann die erste und / oder die zweite Komponente des Mehrkomponentensystem jeweils ein Treibgas umfassen, wobei das Treibgas insbesondere aus der Gruppe Dimethylether, Alkanen, wie Propan, n-Butan, iso-Butan, sowie Mischungen von diesen ausgewählt sein kann. Ein derartiges Mehrkomponentensystem eignet sich, wie bereist weiter oben dargelegt, besonders gut zur Herstellung von Schäumen, insbesondere zur Wundbehandlung.

Gegenstand der Erfindung ist auch die Verwendung eines erfindungsgemäßen Mehrkomponentensystems zur Herstellung eines Schaums, insbesondere zur Behandlung von Wunden.

Ein weiterer Gegenstand der vorliegenden Erfindung betrifft einen Formkörper, insbesondere in Form einer Wundauflage, der durch Vermischen der ersten und zweiten Komponente eines erfindungsgemäßen isocyanatfreien Mehrkomponentensystems und vollständiger Polymerisation der hierbei entstehenden Mischungen erhältlich ist. Der erfindungsgemäße Formkörper kann geschäumt oder ungeschäumt sein. Bevorzugt handelt es sich jedoch um einen geschäumten Formkörper.

Bevorzugt polymerisiert die Mischung bei Raumtemperatur in einem Zeitraum von maximal fünf Minuten vollständig, bevorzugt innerhalb von drei Minuten, weiter bevorzugt innerhalb von einer Minute.

Unter vollständiger Polymerisation wird im Sinne der vorliegenden Erfindung verstanden, dass nicht nur eine äußerliche Hautbildung erfolgt, mit anderen Worten, dass die Außenhülle des Formkörpers nicht mehr klebrig ist, sondern dass die Präpolymere weitestgehend vollständig abreagiert sind. Dies wird im Rahmen der vorliegenden Erfindung in der Weise überprüft, dass der hergestellte Formkörper mit einem Finger für einige Sekunden vollständig eingedrückt wird und anschließend bei Wegnahme des Fingerdrucks von selbst in die Ausgangslage zurückkehrt.

Eine derart schnelle Aushärtung ist insbesondere bei medizinischen Anwendungen von Vorteil, speziell bei der Verwendung als Wundauflage. Denn erst durch die äußerst schnelle Härtung kann die Wundauflage zeitnah einbandagiert werden und vom Patienten mechanisch belastet werden. Dadurch können lange Wartezeiten vermieden werden.

Ein weiterer Gegenstand der Erfindung ist die Verwendung eines insbesondere geschäumten Formkörpers als Wundauflage. Eine solche Wundauflage hat den Vorteil, dass durch die Schaumstruktur nicht nur Wundsekrete aufgenommen werden können, sondern hierdurch gleichzeitig ein mechanischer Schutz der Wunde vor Stößen und dergleichen erzielt wird. Auch der Druck von Kleidungsstücken auf der Wunde wird durch die Schaumstruktur teilweise aufgenommen.

Des Weiteren passt sich die gesprühte Wundauflage ideal den zumeist unregelmäßigen Konturen einer Wunde an, gewährleistet somit eine Wundabdeckung weitestgehend frei von Druckschmerzen, welche durch unpassende Wundauflage verursacht werden. Zusätzlich verkürzt die erfindungsgemäß hergestellte Wundauflage den für die Wundversorgung benötigten Zeitaufwand im Vergleich zu einer Versorgung mit einer traditionellen Wundauflage, da keine Anpassung mittels zeitaufwendigen Zuschnitts erforderlich ist.

Die vorliegende Erfindung betrifft weiterhin eine Mehrkammerdruckdose mit einem Auslassventil und einer Mischdüse, enthaltend ein erfindungsgemäßes Mehrkomponentensystem, wobei die erste und zweite Komponente des Mehrkomponentensystems getrennt in einer ersten und einer zweiten Kammer der Mehrkammerdruckdose eingefüllt und wenigstens eine der Kammern mit einem Flüssigtreibgas unter Überdruck beaufschlagt ist, insbesondere mit einem Druck von wenigstens 1,5 bar. Das Flüssigtreibgas in den Kammern kann gleich oder verschieden sein. Eine für diesen Zweck besonders geeignete Zweikammerdruckdose ist beispielsweise aus den noch nicht veröffentlichten PCT Anmeldungen mit den Anmeldenummern PCT/EP2011/063910 und PCT/EP2011/063909 bekannt, deren Inhalt in die vorliegende Anmeldung vollständig einbezogen wird.

Nach einer weiteren Ausführungsform der erfindungsgemäßen Mehrkammerdruckdose sind die Treibgase sowohl in der ersten als auch in der zweiten Komponente löslich, wobei die Löslichkeit bei einem Fülldruck von mindestens 1,5 bar und bei einer Temperatur von 20°C wenigstens 3 Gew.-% beträgt und wobei insbesondere höchstens so viel Treibgas eingefüllt ist, wie der Löslichkeit entspricht. Auf diese Weise wird dafür gesorgt, dass der ausgesprühte Schaum eine gleichbleibende Qualität besitzt, weil nicht aus einer der Kammern zu Beginn des Sprühvorgangs nur Treibgas entweicht und damit das Mischungsverhältnis zwischen erster und zweiter Komponente nicht optimal ist.

Ein weiterer Vorteil ist darin begründet, dass aufgrund der Löslichkeit des Treibgases in den Kammern der Druckdose keine Phasenseparation zwischen erster bzw. zweiter Komponente und dem Treibgas entsteht. Das Treibgas entweicht deshalb erst beim Auslösen der Druckdose und Vermischen der ersten und zweiten Komponente und schäumt diese Mischung dabei auf. Die sehr schnelle Aushärtungszeit der erfindungsgemäßen Mehrkomponentensysteme führt dazu, dass die durch das Treibgas aufgeschäumte Schaumstruktur "eingefroren" wird und nicht in sich zusammenfällt.

Der vorgenannte Effekt wird durch den Einsatz der erfindungsgemäß vorgesehenen Polyurethandispersion (zweite Komponente) verstärkt, da die Dispersion in gewissem Maße stabilisierende Eigenschaften auf den Schaum hat. Eine Treibgaslöslichkeit von wenigstens 3 Gew.-% ist von Vorteil, um ein ausreichendes Aufschäumen der ausgebrachten Mischung sicherzustellen. Vorzugsweise enthält die erste Komponente einen Gehalt von 10 bis 40 Gew.-% Treibgas, besonders bevorzugt 15 bis 25 Gew.-% und die zweite Komponente einen Gehalt von3 bis 20 Gew.-% Treibgas, besonders bevorzugt 5 bis 15 Gew.-% jeweils bezogen auf das resultierende Gesamtgewicht der jeweiligen Mischung. Hierbei kann durch die Menge an eingefüllter bzw. in den einzelnen Komponenten gelöster Treibgasmenge auch die Schaumstruktur beeinflusst werden. So führt bei einer Zusammensetzung eine höhere Treibgasmenge in der Regel zu einem Schaum geringerer Dichte.

In besonders bevorzugter Weise ist das Treibgas ausgewählt aus Dimethylether, Alkanen, wie Propan, n-Butan, iso-Butan, sowie Mischungen von diesen. Diese Treibgase sind besonders vorteilhaft, weil sich herausgestellt hat, dass diese sowohl in der ersten Komponente, die das Silan-Präpolymer enthält, als auch in der zweiten Komponente, der wässrigen Polyurethandispersion, löslich sind. Von den vorgenannten Treibgasen sind die Alkane ganz besonderes bevorzugt, die diese im Gegensatz zu Dimethylether bei Kontakt mit offenen Wunden ein weniger brennendes Gefühl beim Patienten hervorrufen.

Ein weiterer Gegenstand der vorliegenden Erfindung betrifft die Verwendung eines erfindungsgemäßen Mehrkomponentensystems zur Herstellung eines geschäumten oder ungeschäumten Formkörpers, insbesondere eines flächigen Formkörpers wie einer Wundauflage.

Die vorliegende Erfindung wird im Folgenden anhand von Ausführungsbeispielen näher erläutert.

### Beispiele:

### Allgemeines:

Alle folgenden Mengenangaben, Anteile und Prozentanteile sind, soweit nicht anders angegeben, auf das Gewicht und die Gesamtmenge bzw. auf das Gesamtgewicht der Zusammensetzungen bezogen.

Sofern nicht abweichend vermerkt, beziehen sich alle analytischen Messungen auf Messungen bei Temperaturen von 23°C.

### Methoden:

Die Feststoff- bzw. Festkörpergehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter bei einer Rotationsfrequenz von 18 s⁻¹ der Firma Anton Paar Germany GmbH, Ostfildern, DE bestimmt.

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

Die Lagerstabilität der Dispersionen wurde über einen Zeitraum von 6 Monaten nach Herstellung durch Lagerung bei Raumtemperatur überprüft.

Die Bruchdehnung wurde bestimmt gemäß DIN 53504.

Die maximal lösliche Treibgasmenge wurde bei 20°C in "Schaugläsern zur optischen Prüfung von Aerosolen" der Firma Pamasol Willi Mäder AG, CH ermittelt. Die maximal lösliche Treibgasmenge bezieht sich auf das Gewichtsverhältnis von Treibgas zur zu untersuchenden Substanz/Mischung und war erreicht, sobald das Treibgas gerade noch dauerhaft (> 1h) keine zweite Phase ausbildete.

Zum Ausschäumen der Mischungen wurde eine 2K-Sprühapparatur verwendet und in der Weise gefüllt, wie in den PCT-Anmeldungen mit den Anmeldenummern PCT/EP2011/063910 und PCT/EP2011/063909 beschrieben ist.

### Verwendete Substanzen und Abkürzungen:

- Diaminosulfonat:: NH₂-CH₂CH₂-NH-CH₂CH₂-SO₃Na (45 %-ig in Wasser)
- HDI:: Hexamethylen-1,6-diisocyanat
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlenmittleres Molekulargewicht 1000 g/mol (BASF AG, Ludwigshafen, DE)
- Desmophen^{®} C 2200:: Lineares, aliphatisches Polycarbonatdiol mit endständigen Hydroxylgruppen und einem Molekulargewicht von ca. 2.000 g/mol, Bayer MaterialScience AG, Leverkusen, DE.
- Polyether LB 25:: Monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE)
- Desmodur^{®} N 3300:: HDI-Trimerisat, NCO-Gehalt 21,8 ± 0,3 Gew.% (Bayer MaterialScience AG, Leverkusen, DE)
- Desmodur^{®} XP 2599:: Aliphatisches, Ethergruppen enthaltendes Präpolymer auf Basis HDI, NCO-Äquivalentgewicht ca. 700 g (Bayer MaterialScience AG, Leverkusen, DE)
- Desmodur^{®} XP 2617:: Weitestgehend lineares NCO-Präpolymer auf Basis HDI, NCO-Gehalt 12,5 ± 1,0 Gew.% (Bayer MaterialScience AG, Leverkusen, DE)
- Geniosil^{®} XL 926:: [(Cyclohexylamino)methyl]triethoxysilan (Wacker Chemie AG, München, DE)
- P/B 3,5:: Mischung aus Propan und iso-Butan, so dass bei 20°C ein Gasdruck von 3,5 bar resultiert
- P/B 4,5:: Mischung aus Propan und iso-Butan, so dass bei 20°C ein Gasdruck von 4,5 bar resultiert
- DME:: Dimethylether

### Beispiel 1: Herstellung der wässrigen Polyurethandispersion PUD1

450 g PolyTHF 1000 und 2100 g PolyTHF 2000 wurden auf 70°C aufgeheizt. Anschließend wurde ein Gemisch aus 225,8 g Hexamethylendiisocyanat und 298,4 g Isophorondiisocyanat zugegeben und solange bei 100-115°C gerührt bis der theoretische NCO-Wert leicht unterschritten war. Das fertige Prepolymer wurde mit 5460 g Aceton bei 50°C gelöst und anschließend eine Lösung aus 29,5 g Ethylendiamin, 143,2 g Diaminosulfonat und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion mit einem Festkörpergehalt von 56 %, einer Partikelgröße von 276 nm und einer Viskosität von 1000 mPas erhalten. In der mit wässriger Zitronensäure auf pH 5,5 eingestellten Dispersion (Festkörpergehalt 48 %) konnten maximal 3,1 % P/B 3,5 gelöst werden.

### Beispiel 2: Herstellung der wässrigen Polyurethandispersion PUD2

1645 g PolyTHF 2000, 352,5 g PolyTHF 1000 und 158,6 g Polyether LB 25 wurden auf 70°C aufgeheizt. Anschließend wurde bei 70°C innerhalb von 5 min ein Gemisch aus 177 g Hexamethylendiisocyanat und 234 g Isophorondiisocyanat zugegeben und solange gerührt bis der theoretische NCO-Wert unterschritten war. Das fertige Prepolymer wurde mit 4560 g Aceton bei 50°C gelöst und anschließend eine Lösung aus 23,1 g Ethylendiamin und 45,2 g Isophorondiamin und 294 g Wasser innerhalb von 10 min zudosiert. Die Nachrührzeit betrug 10 min. Danach wurde innerhalb von 10 min durch Zugabe von 1650 g Wasser dispergiert. Es folgte die Entfernung des Lösemittels durch Destillation im Vakuum und es wurde eine lagerstabile Dispersion mit einem Festkörpergehalt von 49 %, einer Partikelgröße von 255 nm und einer Viskosität von 420 mPas erhalten. In der mit Wasser auf einen Festkörpergehalt von 30 % eingestellten Dispersion konnten maximal 20,5 % P/B 4,5 gelöst werden.

### Beispiel 3: Herstellung der wässrigen Polyurethandispersion PUD3

174,3 g Desmophen C 2000, 37,3 g PolyTHF 1000, 3,9 g Neopentylglykol, 34,6 g Polypropylenglykol-monobutylether mit einer zahlenmittleren Molmasse von 2500 g/mol und 18,9 g Polyether LB 25 wurden auf 70°C aufgeheizt. Anschließend wurde bei 70°C innerhalb von 5 min ein Gemisch aus 23,2 g Hexamethylendiisocyanat und 30,7 g Isophorondiisocyanat zugegeben und solange gerührt bis der theoretische NCO-Wert unterschritten war. Danach werden bei 70°C 4,4 g N-Methyldiethanolamin zugesetzt. Das fertige Prepolymer wurde mit 330 g Aceton bei 50°C gelöst und anschließend eine Lösung aus 4,7 g Isophorondiamin in 8,4 g Aceton und nach 5 Minuten 0,1 g Ethylendiamin in 0,6 g Wasser zudosiert. Die Nachrührzeit betrug 10 min. Danach wurden 35,3 g 1 normale Salzsäure zugesetzt und nach weiteren 5 Minute innerhalb von 10 min durch Zugabe von 740 g Wasser dispergiert. Es folgte die Entfernung des Lösemittels durch Destillation im Vakuum und es wurde eine lagerstabile Dispersion mit einem Festkörpergehalt von 32 %, einer Partikelgröße von 273 nm und einer Viskosität von < 50 mPas erhalten. In der Dispersion konnten maximal 21,1 % P/B 3,5 gelöst werden.

### Beispiel 4: Herstellung der wässrigen Polyurethandispersion PUD4

159,4 g PolyTHF 2000, 3,9 g Neopentylglykol, 34,6 g Polypropylenglykol-monobutylether mit einer zahlenmittleren Molmasse von 2500 g/mol und 118,9 g Polyether LB 25 wurden auf 70°C aufgeheizt. Anschließend wurde bei 70°C innerhalb von 5 min ein Gemisch aus 28,2 g Hexamethylendiisocyanat und 37,3 g Isophorondiisocyanat zugegeben und solange gerührt bis der theoretische NCO-Wert unterschritten war. Danach werden bei 70°C 4,4 g N-Methyldiethanolamin zugesetzt. Das fertige Prepolymer wurde mit 390 g Aceton bei 50°C gelöst und anschließend eine Lösung aus 4,9 g Isophorondiamin in 8,7 g Aceton und nach 5 Minuten 0,1 g Ethylendiamin in 0,6 g Wasser zudosiert. Die Nachrührzeit betrug 10 min. Danach wurden 35,3 g 1 normale Salzsäure zugesetzt und nach weiteren 5 Minute innerhalb von 10 min durch Zugabe von 880 g Wasser dispergiert. Es folgte die Entfernung des Lösemittels durch Destillation im Vakuum und es wurde eine lagerstabile Dispersion mit einem Festkörpergehalt von 30 %, einer Partikelgröße von 149 nm und einer Viskosität von < 50 mPas erhalten. In der Dispersion konnten maximal 27,1 % P/B 3,5 gelöst werden.

### Beispiel 5: Herstellung der wässrigen Polyurethandispersion PUD5

70,0 g PolyTHF 1000, 326,7 g PolyTHF 2000, 22,9 g Neopentylglykol und 42,7 g Polyether LB 25 wurden auf 70°C aufgeheizt. Anschließend wurde bei 70°C innerhalb von 5 min ein Gemisch aus 53,8 g Hexamethylendiisocyanat und 71,1 g Isophorondiisocyanat zugegeben und solange gerührt bis der theoretische NCO-Wert unterschritten war. Danach wurden bei 70°C 9,2 g N-Methyldiethanolamin zugesetzt. Das fertige Prepolymer wurde mit 600 g Aceton bei 50°C gelöst und anschließend eine Lösung aus 13,7 g Isophorondiamin in 24,3 g Aceton zudosiert. Die Nachrührzeit betrug 10 min. Danach wurden 73,2 g 1 normale Salzsäure zugesetzt und nach weiteren 5 Minute innerhalb von 10 min durch Zugabe von 1350 g Wasser dispergiert. Es folgte die Entfernung des Lösemittels durch Destillation im Vakuum und es wurde eine lagerstabile Dispersion mit einem Festkörpergehalt von 30 %, einer Partikelgröße von 301 nm und einer Viskosität von < 50 mPas erhalten.

Die folgenden Beispiele zeigen die Herstellung der silan-terminierten Präpolymere.

### Beispiel 6: Herstellung des silan-terminierten Präpolymers STP1

Zu einem Gemisch aus 1000 g HDI und 1 g Benzoylchlorid wurde bei 80°C innerhalb von 3h 1000 g eines Polyalkylenoxids mit einer Molmasse von 4680 g/mol gestartet auf Glycerin, einem Ethylenoxidgewichtsanteil von 71 % und Propylenoxidgewichtsanteil von 26 %, das zuvor bei 100°C während 6h bei einem Druck von 0,1 mbar getrocknet wurde, zugetropft und für 12h nachgerührt. Das überschüssige HDI wurde durch Dünnschichtdestillation bei 130°C und 0,1 mbar entfernt. Man erhielt ein Präpolymer mit einem NCO-Gehalt von 2,42 % und einer Viskosität von 3500 mPas.

200 g des erhaltenen Präpolymers wurden anschließend bei 30-40°C innerhalb von 10 Minuten mit 31,7 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Es wurde eine viskose, farblose Flüssigkeit erhalten.

### Beispiel 7: Herstellung des silan-terminierten Präpolymers STP2

Zu 390 g Desmodur N 3300 wurden bei 80°C 1125 g eines Polyalkylenoxids mit einer Molmasse von 2250 g/mol gestartet auf Butyldiglykol, einem Ethylenoxidgewichtsanteil von 79 % und Propylenoxidgewichtsanteil von 14 %, das zuvor bei 100°C während 2h bei einem Druck von 0,1 mbar getrocknet wurde, zugetropft und bis zum Erreichen eines NCO-Gehalts von 3,67% bei 80°C nachgerührt.

5,0 g des erhaltenen Präpolymers wurden anschließend bei Raumtemperatur innerhalb von 10 Minuten mit 1,2 g Geniosil XL 926 versetzt. Nach weiteren 30 Minuten Rühren konnte die vollständige Umsetzung des NCO Präpolymers zum STP IR-spektroskopisch nachgewiesen werden. Es wurde eine viskose, farblose Flüssigkeit erhalten.

### Beispiel 8: Herstellung des silan-terminierten Präpolymers STP3

Zu 490 g Desmodur XP 2599 wurden bei 80°C 394 g eines Polyalkylenoxids mit einer Molmasse von 2250 g/mol gestartet auf Butyldiglykol, einem Ethylenoxidgewichtsanteil von 79 % und Propylenoxidgewichtsanteil von 14 %, das zuvor bei 100°C während 2h bei einem Druck von 0,1 mbar getrocknet wurde, zugetropft und bis zum Erreichen eines NCO-Gehalts von 2,22% bei 80°C nachgerührt.

Eine Lösung von 51,8 g des erhaltenen Präpolymers in 70 g trockenem Diethylether wurde anschließend bei Raumtemperatur innerhalb von 10 Minuten mit 7,5 g Geniosil XL 926 versetzt. Nach weiteren 30 Minuten Rühren konnte die vollständige Umsetzung des NCO Präpolymers zum STP IR-spektroskopisch nachgewiesen werden. Es wurde eine viskose, farblose Flüssigkeit erhalten.

### Beispiel 9: Herstellung des silan-terminierten Präpolymers STP4

Eine Lösung von 50 g Desmodur XP 2617 in 55 g trockenem Aceton wurde bei 30°C innerhalb von 30 Minuten mit 42 g Geniosil XL 926 versetzt. Nach weiteren 30 Minuten Rühren bei 40°C konnte die vollständige Umsetzung des NCO Präpolymers zum STP IR-spektroskopisch nachgewiesen werden. Es wurde eine viskose, farblose Flüssigkeit erhalten.

### Beispiel 10: Herstellung des silan-terminierten Präpolymers STP5

Zu einem Gemisch aus 800 g eines Polyalkylenoxids mit einer Molmasse von 2000 g/mol gestartet auf 1,2-Propylenglykol, einem Ethylenoxidgewichtsanteil von 47 % und Propylenoxidgewichtsanteil von 49 %, das zuvor bei 80°C während 1h bei einem Druck von 0,1 mbar getrocknet wurde, und 2,8 g Benzoylchlorid wurde bei 80°C innerhalb von 45 Minuten 1000 g HDI zugetropft und für 2h nachgerührt. Das überschüssige HDI wurde durch Dünnschichtdestillation bei 130°C und 0,4 mbar entfernt. Man erhielt ein Präpolymer mit einem NCO-Gehalt von 3,43 % und einer Viskosität von 1250 mPas.

498 g des erhaltenen Präpolymers wurden anschließend bei 30-40°C innerhalb von 15 Minuten mit 104,5 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Das resultierende STP wies gegenüber dem eingesetzten NCO-Präpolymer eine höhere Viskosität auf.

### Beispiel 11: Herstellung des silan-terminierten Präpolymers STP6

Zu einem Gemisch aus 1032 g eines Polyalkylenoxids mit einer Molmasse von 4000 g/mol gestartet auf 1,2-Propylenglykol, einem Ethylenoxidgewichtsanteil von 13 % und Propylenoxidgewichtsanteil von 86 %, das zuvor bei 80°C während 1h bei einem Druck von 0,1 mbar getrocknet wurde, und 1,8 g Benzoylchlorid wurde bei 80°C innerhalb von 30 Minuten 650 g HDI zugetropft und für 4h nachgerührt. Das überschüssige HDI wurde durch Dünnschichtdestillation bei 130°C und 0,03 mbar entfernt. Man erhielt ein Präpolymer mit einem NCO-Gehalt von 1,82 % und einer Viskosität von 2100 mPas.

207,5 g des erhaltenen Präpolymers wurden anschließend bei 30-40°C innerhalb von 15 Minuten mit 24,8 g Geniosil XL 926 versetzt. Nach weiteren 30 Minuten Rühren bei 30°C konnte die vollständige Umsetzung des NCO Präpolymers zum STP IR-spektroskopisch nachgewiesen werden. Das resultierende STP wies gegenüber dem eingesetzten NCO-Präpolymer eine höhere Viskosität auf.

### Beispiel 12: Herstellung des silan-terminierten Präpolymers STP7

Zu einem Gemisch aus 398 g eines Polyalkylenoxids mit einer Molmasse von 4800 g/mol gestartet auf Glycerin, einem Ethylenoxidgewichtsanteil von 13 % und Propylenoxidgewichtsanteil von 85 %, das zuvor bei 80°C während 1h bei einem Druck von 0,1 mbar getrocknet wurde, und 0,7 g Benzoylchlorid wurde bei 80°C innerhalb von 30 Minuten 315 g HDI zugetropft und für 2h nachgerührt. Das überschüssige HDI wurde durch Dünnschichtdestillation bei 140°C und 0,07 mbar entfernt. Man erhielt ein Präpolymer mit einem NCO-Gehalt von 2,10 %.

200 g des erhaltenen Präpolymers wurden anschließend bei 30-40°C innerhalb von 10 Minuten mit 27,6 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Es wurde eine viskose, farblose Flüssigkeit erhalten.

### Beispiel 13: Herstellung des silan-terminierten Präpolymers STP8

Zu einem Gemisch aus 201 g eines Polyalkylenoxids mit einer Molmasse von 1000 g/mol gestartet auf 1,2-Propylenglykol und einem Propylenoxidgewichtsanteil von 92 %, das zuvor bei 80°C während 1h bei einem Druck von 0,1 mbar getrocknet wurde, und 0,8 g Benzoylchlorid wurde bei 80°C innerhalb von 30 Minuten 588 g HDI zugetropft und für 2h nachgerührt. Das überschüssige HDI wurde durch Dünnschichtdestillation bei 140°C und 0,05 mbar entfernt. Man erhielt ein Präpolymer mit einem NCO-Gehalt von 6,09 %.

200 g des erhaltenen Präpolymers wurden anschließend bei 30-40°C innerhalb von 10 Minuten mit 80 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Es wurde eine viskose, farblose Flüssigkeit erhalten.

### Beispiel 14: Herstellung des silan-terminierten Präpolymers STP9

Zu einem Gemisch aus 189 g eines Polyesterpolyols basierend auf Diethylenglykol und Adipinsäure mit einer Molmasse von 1000 g/mol, das zuvor bei 80°C während 30 min bei einem Druck von 5 mbar getrocknet wurde, und 0,9 g Benzoylchlorid wurde bei 70-80°C innerhalb von 40 Minuten 477 g HDI zugetropft und für 2h nachgerührt. Das überschüssige HDI wurde durch Dünnschichtdestillation bei 140°C und 0,05 mbar entfernt. Man erhielt ein Präpolymer mit einem NCO-Gehalt von 5,81 % und einer Viskosität von 6100 mPas.

160 g des erhaltenen Präpolymers wurden anschließend bei 30-40°C innerhalb von 15 Minuten mit 61 g Geniosil XL 926 versetzt. Nach weiteren 30 Minuten Rühren bei 30°C konnte die vollständige Umsetzung des NCO Präpolymers zum STP IR-spektroskopisch nachgewiesen werden. Es wurde eine viskose, farblose Flüssigkeit erhalten.

### Beispiel 15: Herstellung des silan-terminierten Präpolymers STP10

Zu einem Gemisch aus 423 g eines Polyalkylenoxids mit einer Molmasse von 3825 g/mol gestartet auf Trimethylolpropan, einem Ethylenoxidgewichtsanteil von 13 % und Propylenoxidgewichtsanteil von 83 %, das zuvor bei 80°C während 1h bei einem Druck von 0,1 mbar getrocknet wurde, und 0,8 g Benzoylchlorid wurde bei 80°C innerhalb von 30 Minuten 420 g HDI zugetropft und für 2h nachgerührt. Das überschüssige HDI wurde durch Dünnschichtdestillation bei 130°C und 0,03 mbar entfernt. Man erhielt ein Präpolymer mit einem NCO-Gehalt von 2,84 %.

200 g des erhaltenen Präpolymers wurden anschließend bei 30-40°C innerhalb von 10 Minuten mit 37 g Geniosil XL 926 versetzt. Nach weiteren 60 Minuten Rühren bei 30°C konnte die vollständige Umsetzung des NCO Präpolymers zum STP IR-spektroskopisch nachgewiesen werden. Es wurde eine viskose, farblose Flüssigkeit erhalten.

### Beispiel 16: Herstellung des silan-terminierten Präpolymers STP11

Zu einem Gemisch von 270 g des gemäß Beispiel 10 hergestellten NCO-Präpolymers und 1349 g des gemäß Beispiel 11 hergestellten NCO-Präpolymers wurden bei 30-40°C innerhalb von 30 Minuten 217 g Geniosil XL 926 zugetropft und für weitere 30 Minuten bei 30°C gerührt. Die vollständige Umsetzung des NCO-Präpolymers zum STP konnte IR-spektroskopisch nachgewiesen werden. Es wurde eine viskose, farblose Flüssigkeit erhalten.

In den folgenden Versuchen werden die Ergebnisse der Härtungsversuche der Schäume dargestellt.

### Beispiel 17: Härtung von STP und PUD als Schaum

In 100 g Präpolymer STP1 wurden 38,6 g DME und 4,3 g P/B 3,5 gelöst.

In 40 g PUD5 wurden 1,2 g P/B 3,5 gelöst.

Die beiden Komponenten wurden einzeln in jeweils eine Kammer einer Pressluft betriebenen 2K-Sprühapparatur gefüllt. Das synchrone Ausbringen beider Komponenten im Volumenverhältnis 2,5 (STP) zu 1 (PUD) erfolgte über einen Statikmischer, worin die Durchmischung erfolgte. Unter Aufschäumen und vollständiger Aushärtung innerhalb von etwa 60 s wurde ein farbloser, elastischer, feinporiger Schaum erhalten.

### Beispiel 18: Härtung von STP und PUD als Schaum

In 100 g Präpolymer STP11 wurden 26,6 g n-Butan gelöst. In 40 g PUD5 wurden 1,2 g P/B 3,5 gelöst.

Die beiden Komponenten wurden einzeln in jeweils eine Kammer einer Pressluft betriebenen 2K-Sprühapparatur gefüllt. Das synchrone Ausbringen beider Komponenten im Volumenverhältnis 2,5 (STP) zu 1 (PUD) erfolgte über einen Statikmischer, worin die Durchmischung erfolgte. Unter Aufschäumen und vollständiger Aushärtung innerhalb von etwa 10 s wurde ein farbloser, elastischer, feinporiger Schaum erhalten.

### Beispiel 19: Härtung von STP und PUD als Schaum

In 100 g Präpolymer STP9 wurden 44,9 g DME gelöst. In 40 g PUD5 wurden 1,2 g P/B 3,5 gelöst.

Die beiden Komponenten wurden einzeln in jeweils eine Kammer einer Pressluft betriebenen 2K Sprühapparatur gefüllt. Das synchrone Ausbringen beider Komponenten im Volumenverhältnis 2,5 (STP) zu 1 (PUD) erfolgte über einen Statikmischer, worin die Durchmischung erfolgte. Unter Aufschäumen und vollständiger Aushärtung innerhalb von etwa 30 s wurde ein farbloser, elastischer Schaum erhalten.

### Beispiel 20: Härtung von STP und PUD als Schaum

In 100 g Präpolymer STP11 wurden 40,8 g P/B 3,5 gelöst. 40 g PUD5 wurden ohne Begasung verwendet.

Die beiden Komponenten wurden einzeln in jeweils eine Kammer einer Pressluft betriebenen 2K-Sprühapparatur gefüllt. Das synchrone Ausbringen beider Komponenten im Volumenverhältnis 2,5 (STP) zu 1 (PUD) erfolgte über einen Statikmischer, worin die Durchmischung erfolgte. Unter Aufschäumen und vollständiger Aushärtung innerhalb von etwa 5 s wurde ein farbloser, elastischer, feinporiger Schaum erhalten.

### Beispiel 21: Härtung von STP und PUD als Schaum

In 100 g Präpolymer STP5 wurden 31,6 g DME gelöst. In 40 g PUD1, welche zuvor mittels Zitronensäure auf einen pH-Wert von 5,5 eingestellt wurde, wurden 3,1 g P/B 3,5 gelöst.

Die beiden Komponenten wurden einzeln in jeweils eine Kammer einer Pressluft betriebenen 2K-Sprühapparatur gefüllt. Das synchrone Ausbringen beider Komponenten im Volumenverhältnis 2,5 (STP) zu 1 (PUD) erfolgte über einen Statikmischer, worin die Durchmischung erfolgte. Unter Aufschäumen und vollständiger Aushärtung innerhalb von etwa 60 s wurde ein elastischer, kompakter Schaum mit verfilmter Oberfläche erhalten.

Diese Beispiele belegen, dass sich ausgehend von verschiedenen Ausgangssubstanzen, d.h. Polyurethandispersionen und Silan-Präpolymeren, eine Vielzahl unterschiedlicher Schäume herstellen lässt. Doch sind die Zusammensetzungen nicht nur zur Herstellung von Schäumen verwendbar, sondern, wie die folgenden Beispiele zeigen, auch als Ausgangsbasis zur Herstellung von Vergussmassen geeignet.

### B

### eispiel 22: Härtung von STP und PUD als Vergussmasse

100 g Präpolymer STP11 und 33 g PUD1 wurden zügig miteinander verrührt und auf Trennpapier mit einer Schichtdicke von etwa 6 mm ausgebracht. Die bereits nach 10 s hochviskose Masse war innerhalb von 30 s vollständig ausgehärtet. Es wurde eine kompakte Gussmasse mit einer Bruchdehnung von 35% erhalten.

### Versteichsbeispiel gemäß EP 1 829 908, Beispiel 1:

Bei diesem Vergleichsversuch soll die erfindungsgemäße Funktion der Polyurethandispersion mit den aus dem Stand der Technik bekannten 2K-Systemen verglichen werden, vorliegend mit dem Beispiel 1 der EP 1 829 908. In Komponente 2 (8 Teile Wasser, 13 Teile Zitronensäure) konnte hierbei kein Treibgas P/B 3,5 gelöst werden. Aufgrund dessen lässt sich diese Zusammensetzung nicht zufriedenstellend aus der 2K-Sprühapparatur ausbringen, da weder die Mischungsverhältnisse zwischen der Präpolymerkomponente und der wässrigen Komponente exakt einstellbar waren, noch eine ausreichende Aufschäumung der Mischung erzielt werden konnte.

## Patentansprüche

1. Isocyanatfreies Mehrkomponentensystem, insbesondere zur Herstellung von Schäumen für medizinische Produkte wie Wundauflagen, mit wenigstens zwei getrennten Komponenten, wobei die erste Komponente zumindest ein Alkoxysilan-terminiertes Präpolymer und die zweite Komponente eine wässrige Komponente umfasst, wobei die wässrige Komponente eine Polyurethandispersion umfasst.

2. Mehrkomponentensystem nach Anspruch 1, **dadurch gekennzeichnet, dass** das Alkoxysilan-terminierte Präpolymer ein Alkoxysilan-terminiertes Polyurethanpräpolymer umfasst, welches insbesondere aus einem Aminoalkoxysilan und einem Isocyanat-terminierten Präpolymer hergestellt ist, wobei das Isocyanat-terminierte Präpolymer insbesondere aus einer Polyolkomponente und einer aliphatischen Isocyanatkomponente hergestellt ist.

3. Mehrkomponentensystem nach Anspruch 2, **dadurch gekennzeichnet, dass** das Alkoxysilan-terminierte Polyurethanpräpolymer ein Polyester- und/ oder Polyetherpolyol aufweist, wobei der Anteil an Ethylenoxid-Bausteinen im Polyetherpolyol insbesondere höchstens 50 Gew.-% beträgt, vorzugsweise höchstens 30 Gew.-%, weiter bevorzugt höchstens 20 Gew.-%.

4. Mehrkomponentensystem nach einem der vorstehenden Ansprüche, **dadurch gekennzeichnet, dass** das Alkoxysilan-terminierte Präpolymer α-Silangruppen umfasst, insbesondere Triethoxy-α-Silangruppen.

5. Mehrkomponentensystem nach einem der vorstehenden Ansprüche, **dadurch gekennzeichnet, dass** das Gewichtsmittel des Alkoxysilan-terminierten Präpolymers 500 bis 20000 g/Mol beträgt, bevorzugt 500 bis 6000 g/Mol, insbesondere 2000 bis 5000 g/Mol.

6. Mehrkomponentensystem nach einem der vorstehenden Ansprüche, **dadurch gekennzeichnet, dass** die Polyurethandispersion 5 bis 65 Gew.-% Polyurethan enthält, insbesondere 20 bis 60 Gew.-%.

7. Mehrkomponentensystem nach einem der vorstehenden Ansprüche, **dadurch gekennzeichnet, dass** das Gewichtsmittel des Polyurethans der Polyurethandispersion 10000 bis 1000000 g/Mol beträgt, insbesondere 20000 bis 200000 g/Mol.

8. Mehrkomponentensystem nach einem der vorstehenden Ansprüche, **dadurch gekennzeichnet, dass** die Polyurethandispersion einen pH-Wert von 5,0 bis 9,0 aufweist und insbesondere wenigstens eine Säure, eine Base oder ein Puffersystem enthält.

9. Mehrkomponentensystem nach einem der vorstehenden Ansprüche, **dadurch gekennzeichnet, dass** die erste und/ oder die zweite Komponente einen medizinischen Wirkstoff enthält, der insbesondere ausgewählt ist aus Substanzen, die unter in vivo-Bedingungen Stickstoffmonoxidfreisetzen, sowie Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

10. Mehrkomponentensystem nach einem der vorstehenden Ansprüche, **dadurch gekennzeichnet, dass** die erste und die zweite Komponente des Mehrkomponentensystems in einem Volumenverhältnis von 10 : 1 bis 1 : 10 zueinander vorliegen, vorzugsweise in einem Volumenverhältnis von 1 : 1 bis 5 : 1, insbesondere 2 : 1 bis 3 : 1, besonders bevorzugt bei etwa 2,5 : 1.

11. Mehrkomponentensystem nach einem der vorstehenden Ansprüche, **dadurch gekennzeichnet, dass** die erste und / oder die zweite Komponente jeweils ein Treibgas umfasst, wobei das Treibgas insbesondere aus der Gruppe Dimethylether, Alkanen, wie Propan, n-Butan, iso-Butan, sowie Mischungen von diesen ausgewählt ist.

12. Formkörper, insbesondere in Form einer Wundauflage, erhältlich durch Vermischen der ersten und zweiten Komponente eines Mehrkomponentensystems nach einem der Ansprüche 1 bis 10 und vollständige Polymerisation der hierbei entstehenden Mischung.

13. Mehrkammerdruckdose mit einem Auslassventil und einer Mischdüse, enthaltend ein Mehrkomponentensystem nach einem der Ansprüche 1 bis 11, wobei die erste und zweite Komponente des Mehrkomponentensystems getrennt in einer ersten und einer zweiten Kammer der Mehrkammerdruckdose eingefüllt und wenigstens eine der Kammern mit einem Treibgas unter Überdruck beaufschlagt sind.

14. Mehrkammerdruckdose nach Anspruch 13, **dadurch gekennzeichnet, dass** die Kammern mit einem Druck von wenigstens 1,5 bar beaufschlagt sind.

15. Mehrkammerdruckdose nach einem der Ansprüche 13 oder 14, **dadurch gekennzeichnet, dass** das Treibgas sowohl in der ersten als auch in der zweiten Komponente löslich ist, wobei die Löslichkeit bei einem Fülldruck von 1,5 bar und bei einer Temperatur von 20°C wenigstens 3 Gew.-% beträgt und wobei insbesondere höchstens so viel Treibgas eingefüllt ist, wie der Löslichkeit entspricht.

16. Mehrkammerdruckdose nach einem der Ansprüche 13 bis 15, **dadurch gekennzeichnet, dass** das Treibgas ausgewählt ist aus Dimethylether, Alkanen, wie Propan, n-Butan, iso-Butan, sowie Mischungen von diesen.

17. Verwendung eines Mehrkomponentensystems nach einem der Ansprüche 1 bis 11 zur Herstellung eines geschäumten oder ungeschäumten Polymer-Formkörpers, bevorzugt eines flächigen Formkörpers und insbesondere einer Wundauflage.

## Claims

1. Isocyanate-free multicomponent system, more particularly for producing foams for medical products such as wound dressings, having at least two separate components, the first component comprising at least one alkoxysilane-terminated prepolymer and the second component comprising an aqueous component, with the aqueous component comprising a polyurethane dispersion.

2. Multicomponent system according to Claim 1, **characterized in that** the alkoxysilane-terminated prepolymer comprises an alkoxysilane-terminated polyurethane prepolymer prepared more particularly from an aminoalkoxysilane and an isocyanate-terminated prepolymer, the isocyanate-terminated prepolymer having been prepared more particularly from a polyol component and an aliphatic isocyanate component.

3. Multicomponent system according to Claim 2, **characterized in that** the alkoxysilane-terminated polyurethane prepolymer comprises a polyesterpolyol and/or polyetherpolyol, the fraction of ethylene oxide units in the polyetherpolyol being more particularly not more than 50 wt%, preferably not more than 30 wt%, more preferably not more than 20 wt%.

4. Multicomponent system according to any of the preceding claims, **characterized in that** the alkoxysilane-terminated prepolymer comprises α-silane groups, more particularly triethoxy-α-silane groups.

5. Multicomponent system according to any of the preceding claims, **characterized in that** the weight average of the alkoxysilane-terminated prepolymer is 500 to 20 000 g/mol, preferably 500 to 6000 g/mol, more particularly 2000 to 5000 g/mol.

6. Multicomponent system according to any of the preceding claims, **characterized in that** the polyurethane dispersion comprises 5 to 65 wt% of polyurethane, more particularly 20 to 60 wt%.

7. Multicomponent system according to any of the preceding claims, **characterized in that** the weight-average of the polyurethane in the polyurethane dispersion is 10 000 to 1 000 000 g/mol, more particularly 20 000 to 200 000 g/mol.

8. Multicomponent system according to any of the preceding claims, **characterized in that** the polyurethane dispersion has a pH of between 5.0 and 9.0 and in particular comprises at least one acid, one base or one buffer system.

9. Multicomponent system according to any of the preceding claims, **characterized in that** the first and/or the second component comprises an active medical ingredient selected more particularly from substances that release nitrogen monoxide under in vivo conditions, and also substances selected from the group of vitamins or provitamins, carotenoids, analgesics, antiseptics, hemostyptics, antihistamines, antimicrobial metals or salts thereof, plant-based wound healing promoter substances or substance mixtures, plant extracts, enzymes, growth factors, enzyme inhibitors, and also combinations thereof.

10. Multicomponent system according to any of the preceding claims, **characterized in that** the first and the second component of the multicomponent system are present in a volume ratio of 10:1 to 1:10 to one another, preferably in a volume ratio of 1:1 to 5:1, more particularly 2:1 to 3:1, more preferably at about 2.5:1.

11. Multicomponent system according to any of the preceding claims, **characterized in that** the first and/or the second component comprises in each case a propellant gas, the propellant gas being selected more particularly from the group of dimethyl ether, alkanes, such as propane, n-butane, and isobutane, and also mixtures of these.

12. Shaped article, more particularly in the form of a wound dressing, obtainable by mixing the first and second components of a multicomponent system according to any of Claims 1 to 10 and fully polymerizing the resultant mixture.

13. Multichamber pressurized can having an outlet valve and a mixing nozzle, containing a multicomponent system according to any of Claims 1 to 11, the first and second components of the multicomponent system being introduced separately in a first chamber and a second chamber of the multichamber pressurized can, and at least one of the chambers being charged with a propellant gas under superatmospheric pressure.

14. Multichamber pressurized can according to Claim 13, **characterized in that** the chambers are charged with a pressure of at least 1.5 bar.

15. Multichamber pressurized can according to either of Claims 13 and 14, **characterized in that** the propellant gas is soluble both in the first component and in the second component, the solubility being at least 3 wt% under a filling pressure of 1.5 bar and at a temperature of 20°C, and the amount of propellant gas introduced being more particularly not more than the amount corresponding to the solubility.

16. Multichamber pressurized can according to any of Claims 13 to 15, **characterized in that** the propellant gas is selected from dimethyl ether, alkanes, such as propane, n-butane, and isobutane, and also mixtures of these.

17. Use of a multicomponent system according to any of Claims 1 to 11 for producing a foamed or unfoamed, shaped polymer article, preferably a shaped sheetlike article and more particularly a wound dressing.

## Revendications

1. Système multicomposant exempt d'isocyanate, en particulier pour la production de mousses pour produits médicaux tels que des pansements, comportant au moins deux composants séparés, le premier composant comprenant au moins un prépolymère à terminaison alcoxysilane et le deuxième composant comprenant un composant aqueux, le composant aqueux comprenant une dispersion de polyuréthane.

2. Système multicomposant selon la revendication 1, **caractérisé en ce que** le prépolymère à terminaison alcoxysilane comprend un prépolymère polyuréthane à terminaison alcoxysilane qui est préparé en particulier à partir d'un aminoalcoxysilane et d'un prépolymère à terminaison isocyanate, le prépolymère à terminaison isocyanate étant préparé en particulier à partir d'un composant polyol et d'un composant isocyanate aliphatique.

3. Système multicomposant selon la revendication 2, **caractérisé en ce que** le prépolymère polyuréthane à terminaison alcoxysilane comporte un polyester- et/ou polyétherpolyol, la proportion de constituants oxyde d'éthylène dans le polyétherpolyol étant en particulier au maximum de 50 % en poids, de préférence au maximum de 30 % en poids, encore mieux au maximum de 20 % en poids.

4. Système multicomposant selon l'une quelconque des revendications précédentes, **caractérisé en ce que** le prépolymère à terminaison alcoxysilane comprend des groupes α-silane, en particulier des groupes triéthoxy-α-silane.

5. Système multicomposant selon l'une quelconque des revendications précédentes, **caractérisé en ce que** la moyenne en poids du prépolymère à terminaison alcoxysilane vaut de 500 à 20 000 g/mole, de préférence de 500 à 6 000 g/mole, en particulier de 2 000 à 5 000 g/mole.

6. Système multicomposant selon l'une quelconque des revendications précédentes, **caractérisé en ce que** la dispersion de polyuréthane contient 5 à 65 % en poids, en particulier 20 à 60 % en poids, de polyuréthane.

7. Système multicomposant selon l'une quelconque des revendications précédentes, **caractérisé en ce que** la moyenne en poids du polyuréthane de la dispersion de polyuréthane vaut de 10 000 à 1 000 000 g/mole, en particulier de 20 000 à 200 000 g/mole.

8. Système multicomposant selon l'une quelconque des revendications précédentes, **caractérisé en ce que** la dispersion de polyuréthane présente un pH de 5,0 à 9,0, et en particulier contient au moins un acide, une base ou un système tampon.

9. Système multicomposant selon l'une quelconque des revendications précédentes, **caractérisé en ce que** le premier et/ou le deuxième composant contient/contiennent une substance active médicale, qui est en particulier choisie parmi des substances qui dans des conditions in vivo libèrent du monoxyde d'azote, ainsi qu'une substance choisie dans le groupe des vitamines ou provitamines, caroténoïdes, analgésiques, antiseptiques, hémostyptiques, antihistaminiques, métaux antimicrobiens ou leurs sels, substances végétales ou mélanges de substances végétales aidant la cicatrisation, extraits végétaux, enzymes, facteurs de croissance, inhibiteurs d'enzymes ainsi que des associations de ceux-ci.

10. Système multicomposant selon l'une quelconque des revendications précédentes, **caractérisé en ce que** le premier et le deuxième composant du système multicomposant sont présents entre eux en un rapport en volume de 10 : 1 à 1 : 10, de préférence en un rapport en volume de 1 : 1 à 5 : 1, en particulier de 2 : 1 à 3 : 1, de façon particulièrement préférée d'environ 2,5 : 1.

11. Système multicomposant selon l'une quelconque des revendications précédentes, **caractérisé en ce que** le premier et/ou le deuxième composant comprend/comprennent chacun un gaz propulseur, le gaz propulseur étant choisi en particulier dans le groupe constitué par l'éther diméthylique, des alcanes, tels que le propane, le n-butane, l'isobutane, ainsi que des mélanges de ceux-ci.

12. Corps moulé, en particulier sous forme d'un pansement, pouvant être obtenu par mélange du premier et du deuxième composant d'un système multicomposant selon l'une quelconque des revendications 1 à 10 et polymérisation complète du mélange qui en résulte.

13. Récipient sous pression à plusieurs chambres, comportant une valve de sortie et une buse de mélange, contenant un système multicomposant selon l'une quelconque des revendications 1 à 11, le premier et le deuxième composant du système multicomposant étant introduits séparément dans une première et une deuxième chambre du récipient sous pression à plusieurs chambres et au moins l'une des chambres étant alimentée en un gaz propulseur sous surpression.

14. Récipient sous pression à plusieurs chambres selon la revendication 13, **caractérisé en ce que** les chambres sont alimentées avec une pression d'au moins 1,5 bar.

15. Récipient sous pression à plusieurs chambres selon l'une quelconque des revendications 13 et 14, **caractérisé en ce que** le gaz propulseur est soluble aussi bien dans le premier que dans le deuxième composant, la solubilité à une pression de remplissage de 1,5 bar et à une température de 20 °C étant d'au moins 3 % et le gaz propulseur étant introduit en particulier au maximum en une quantité telle qu'elle correspond à la solubilité.

16. Récipient sous pression à plusieurs chambres selon l'une quelconque des revendications 13 à 15, **caractérisé en ce que** le gaz propulseur est choisi parmi l'éther diméthylique, des alcanes, tels que le propane, le n-butane, l'isobutane, ainsi que des mélanges de ceux-ci.

17. Utilisation d'un système multicomposant selon l'une quelconque des revendications 1 à 11, pour la production d'un corps moulé polymère expansé ou non expansé, de préférence d'un corps moulé plat et en particulier d'un pansement.
